# EUROPEAN PATENT SPECIFICATION

(11) **EP 4 478 982 B1**
(45) Date of publication and mention of the grant of the patent: **24.12.2025**
(21) Application number: 23701807.2
(22) Date of filing: 30.01.2023
(51) Int. Cl.: A61C 7/08, A61C 19/06

(54) **COMPUTER-IMPLEMENTED METHOD FOR PRODUCING A SERIES OF DENTAL APPLIANCE FOR INTRA-ORAL DELIVERY OF ONE OR MORE AGENTS**
COMPUTERIMPLEMENTIERTES VERFAHREN ZUR HERSTELLUNG EINER REIHE VON DENTALGERÄTEN ZUR INTRAORALEN ABGABE EINES ODER MEHRERER WIRKSTOFFE
PROCÉDÉ MIS EN OEUVRE PAR ORDINATEUR POUR PRODUIRE UNE SÉRIE D'APPAREILS DENTAIRES POUR L'ADMINISTRATION INTRA-ORALE D'UN OU DE PLUSIEURS AGENTS

(30) Priority: 15.02.2022 EP 22156802
(43) Date of publication of application: 25.12.2024
(73) Proprietor: Bussmedical AG, 6060 Sarnen (CH)
(72) Inventor: OSMANI, Bekim, 4056 Basel (CH); TÖPPER, Tino, 79115 Freiburg (DE); DARD, Elise, 4055 Basel (CH)
(74) Representative: Mertzlufft-Paufler, Cornelius
(86) International application number: PCT/EP2023/052198
(87) International publication number: WO 2023/156181

(56) References cited:
- WO-A1-02/24100
- US-A- 6 126 443
- US-A1- 2009 117 507
- US-A1- 2021 353 387

## Description

The present invention concerns a computer implemented method for producing a serious of different dental appliances.

The disclosure is therefore related to dental appliances to be worn on teeth for intra-oral delivery of one or more agents to a patient, which may be obtained by applying said computer implemented method. Such dental appliances may comprise a solid core, which may be preferably made of a thermoplastic material that can be thermoformed. In particular, the appliance may be fabricated by forming the core into a patient-specific shape.

The disclosure is also related to a series of such appliances, process details for fabricating a dental appliance and a method for reloading (i.e., re-equipping) such an appliance with a releasable agent, for example a flavor, a drug, or an antimicrobial agent.

It is well-known that releasable agents such as antimicrobial agents can be loaded by diffusion processes into porous superficial layers of a dental appliance. When the user is wearing such an appliance in his mouth, the agent may be released from the appliance to provide protection against inflammation of the teeth and gums. Loading such agents into a dental appliance can also be beneficial for keeping the appliance hygienically clean, i.e., free from dangerous pathogens.

WO 02/24100 A1 and US 2009/0117507 A1 both report on respective dental appliances featuring reservoirs for holding and dispensing therapeutic agents during orthodontic treatment.

US 6 126 443 A reports on another dental appliance that can provide controlled release of medication to target dental structures in the mouth.

And US 2021/353387 A1 reports on yet another dental appliance with microstructures extending outwards from the surface and comprising a releasable compound.

Starting out from this background, the invention aims at providing dental appliances and associated methods that can provide substantial benefits for the end-user. In particular, it is an object of the invention to provide means that allow personalized delivery of an agent to a patient's mouth that is tailored to the specific needs of that patient.

The invention is as defined in the appended claims.

For enabling rapid delivery of a dental appliance that is personalized to the specific (in particular medical) needs of a patient, the invention proposes a computer-implemented method for producing (i.e. fabricating) a series of different dental appliances. This method, which is detailed in claim 1, makes use of manufacturing means for producing a series of m different dental appliances and comprises the following steps:
Step (1): specifying a number of m different numerical values for at least one agent release parameter for a particular releasable agent (to be delivered by the appliance to a patient) in a digital data set. Such an "agent release parameter" may be, for example, (a) a maximum initial release rate in mg/hour and/or (b) a total maximum release amount in mg over a time of six hours and/or (c) a maximum areal release rate in (mg/cm²)/hour.
Step (2): calculating a set of m different design parameters for defining a set of m different micro-reservoir-arrangements; this calculation may be advantageously performed by a computer. Moreover, the calculation may be done using a numerical and/or empirical model. Such a model can be derived for example from pre-experiments in which the release rate and/or total amount of releasable agent per micro-reservoir have been measured for a particular micro-reservoir design.

The simplest way of fine-tuning the release rate of a particular appliance can be to alter the spatial density and/or number of micro-reservoirs per appliance within the series of appliances.

Further possible design parameters that define the micro-reservoir arrangement may be: diameter and/or depth of each micro-reservoir; volume of each reservoir; spatial density of the reservoirs; diameter of release hole (if employed); choice of material and thickness of cap layer (if employed); etc. The design parameter characterizing one of the m different micro-reservoir-arrangements can also be a set of different design parameters (for example: location and size of the arrangement; number of micro-reservoirs, volume of each reservoir; density of the reservoirs within the arrangement; etc.)

Step (3): producing (i.e. fabricating) said series of m different dental appliances, wherein each of the m dental appliances features a micro-reservoir-arrangement according to one of the m different design parameters. This production / fabrication is done using said manufacturing means, which will be described in more detail below. In other words, each of the m micro-reservoir arrangements may be different and tailored for the need of a patient at a certain point in time.

As a result, each of the m different dental appliances of that series can meet the respective specified release parameter (as documented in the digital data set).

Another way to meet desired release parameters is to tailor the concentration of the respective agent (that is later to be released from the dental appliance when placed in the mouth of the patient) as contained in a reload-liquid (sometimes described as a "care solution"). Such a reload-liquid may be used to initially load an agent into the dental appliance and/or to replenish/re-load the agent into the dental appliance.

Therefore (in addition or as an alternative to calculating m different design parameters for defining a set of m different micro-reservoir-arrangements in step 2), the method introduced above may be designed such that in step 2 of the method, at least one ingredient parameter for a reload-liquid is calculated. This calculation may be done, for example, for a given set of design parameters specifying the arrangement of micro-reservoirs of the dental appliance to be used with said reload-liquid. When using this dental appliance featuring well-defined micro-reservoirs with a reload-liquid fulfilling said at least one ingredient parameter, the desired release rate may be achieved. In other words, the method can thus deliver patient specific ingredient parameters.

As a result, for each patient, a corresponding customized reload-liquid may be synthesized/mixed according to said at least one ingredient parameter calculated with the computer-implemented method. The customized reload-liquid obtained with the method and a corresponding dental appliance may then be sent to a patient as a product package to be used together. If the dosage is to be adjusted over some time, one or two or even more customized reload-liquids, each with customized concentration of the respective agent, may be produced according to said method (and later sent to the customer) to be used with the same type of dental appliance.

In this method, a computer may be employed to process large data sets and to automatically calculate and deliver the necessary design parameters for defining the micro-reservoirs of each appliance of the series and/or for calculating and delivering the necessary ingredient parameters. The design parameters can also be used as digital input to be delivered to a machine (e.g. a laser device) that forms the micro-reservoirs in the respective cores, for example by ablation. Likewise, the ingredient parameter may be fed to a machine capable of delivering a customized reload-liquid with well-defined concentration of the agent. Such a machine may be an automated liquid mixing device.

It is also possible to fabricate the micro-reservoirs by thermoforming or other forming processes, by ablative processes or by additive manufacturing techniques such as 3D-printing, as has been already described above. Consequently, the method may also be applied to and make use of suitable manufacturing means such as: means for thermoplastic formation of the micro-reservoirs (this may be a hot embossing tool for example); and/or means for ablative formation of the micro-reservoirs (e.g. a laser machining device); and/or means for additive formation of the micro-reservoirs (e.g. a 3D-printer). All of these means, which may also be used in combination, can be used/controlled according to the design parameters calculated with the computer-implemented method.

In other words, said series of m different dental appliances may be produced using dedicated manufacturing means, in particular as detailed above. Moreover, by applying the method, each of the dental appliances may be fabricated according to a specific set of m different design parameters using said manufacturing means.

For each appliance of the series, the generation of the respective specific micro-reservoir arrangement (in 2D or 3D) can comprise locating the respective micro-reservoirs in a specific ROI that has been specified beforehand. The computer-implemented method can thus deliver a digital data set specifying the micro-reservoir-arrangement, e.g., the pattern of arrangement (and hence spatial density) of the micro-reservoirs and/or specific geometrical design parameters, such as a spatial density and/or an average depth and/or a respective volume, of the individual micro-reservoirs of the arrangement for each appliance of the series. Furthermore, the digital data set can also comprise a patient specific location, defined as the ROI, on the respective dental appliance to be fabricated with the method.

These data sets can then be used for fabricating the different appliances, in particular using an ablative process such as laser ablation and/or thermoforming and/or an additive process, for defining the micro-reservoirs in or on the core, in particular in a core foil from which the core is thermoformed.

Following this approach, one embodiment of this method proposes that the digital data set (which specifies the m different numerical values for the at least one agent release parameter) comprises: (i) a set of patient-specific values, each being based on the specific medical need of a single patient, in particular as specified by a treatment instruction for that patient; and/or (ii) a subset of N treatment-specific-values, each based on a specified dosage of the at least one agent to be delivered to a particular patient within a specified time interval.

Such values, for example a total daily dosage to be delivered to a particular patient over a duration of several weeks, may be laid out in a treatment plan for that patient (for example by a doctor treating the patient). For example, by wearing (and reloading) a first dental appliance according to the invention and produced with the computer-implemented method daily over a first period of time, the patient can safeguard that he receives a desired first dosage. After some weeks, the patient may switch to a second dental appliance of the series which offers a lower second dosage of the agent, and he may wear and reload this second appliance repeatedly over a second period of time. Following this concept, the dosage delivered to the patient can be accurately controlled stepwise with minimal effort for the patient, as he only needs to wear/exchange the right appliance designed and produced for the dosage to be delivered within a specific time interval. In this first scenario, a single patient can thus alter the dosage delivered to his mouth by changing the appliances (taken from said series) which he wears on his teeth, and thus conveniently follow a treatment plan that has been recommended by a doctor, for example.

In a second scenario, the dosage to be delivered to a number of m patients can be specified and the method can help in rapidly delivering custom-made and personalized dental appliances for each of those patients that meet the requirements concerning release rate and dosage with respect to a particular agent to be delivered to a specific patient.

The computer-implemented method proposed here thus uses input data such as patient-specific treatment instructions or treatment plans to derive a suitable (digital) data set of design parameters that can then be used to fabricate a series of dental appliances, which can deliver the agent of interest at the desired dosage to a specific patient during the time interval, in which the patient is wearing the appliance in his mouth (e.g. for several hours over night).

In the following, the process for fabricating dental appliances as described herein, which may be part of the method described before, will be detailed:
For example, the fabrication process may be characterized in that a core of the dental appliance is first formed as a core foil, a multitude of micro-reservoirs is/are defined in an outer surface of the core (foil) using: (i) an ablative process, preferably an ablative laser-process, which removes the material of the core at the location of each micro-reservoir; and/or (ii) a forming process, such as hot embossing; and/or (iii) an additive process, for example molding or 3D-printing of an additional outer layer of the core. Of course, it is highly advantageous if the micro-reservoirs defined by that process have features as described before or as specified in the claims directed towards a dental appliance.

The final dental appliance may then be obtained by thermoforming the core (foil) with the already pre-formed micro-reservoirs. Additionally, the final dental appliance may be trimmed in size afterwards, as is known in the art.

Furthermore, as has been explained in detail before, the micro-reservoirs may be filled with a common filling material that is capable of absorbing and releasing an agent such as a flavor molecule or a drug. This may be done prior or after thermoforming of the appliance.

In addition, a cap layer may be deposited onto the core that covers the micro-reservoirs. This may also be done either prior or after the thermoforming of the appliance. As mentioned before, the cap layer may close the micro-reservoirs and/or it may form a diffusion barrier (thereby slowing down diffusion) or it may even be a layer that is impermeable for the agent (thus preventing any diffusion through the cap layer).

Finally, for each micro-reservoir, a corresponding release hole may be formed in the cap layer. This may, preferably, be done using an ablative laser process. This approach is particularly suited when the cap layer is impermeable for the agent.

The laser cutting of the release holes may be done by considering dedicated alignment marks / fiducials; the latter may be patterned together with the micro-reservoirs into or onto the core foil, more precisely into a master foil from which pieces may be cut out to serve as the core foil. Using this approach, each release hole can be accurately positioned over the respective micro-reservoir.

The micro-reservoirs may be patterned into or onto the core with a constant depth and/or volume, preferably such that each micro-reservoir produces the same release-rate and/or release-dosage of the agent in an oral cavity when fully loaded with the agent. Preferably, the multitude of micro-reservoirs may be only defined in at least one spatially limited region of interest (ROI). The at least one ROI may cover less than 10% or even less of an outer surface of the final dental appliance, for example.

The present disclosure also discloses improving the ease of use of the dental appliances described so far. For this purpose, a method is proposed for reloading a dental appliance (which may have features as described before) with a releasable agent such as a flavor, a drug, or an antimicrobial agent.

The method is characterized in that the dental appliance is immersed in a reload-liquid containing the agent. Different from the state-of-the-art, however, the reload-liquid is heated to an elevated temperature of at least 55°C, while the dental appliance is immersed in the reload-liquid.

An electrically heatable reload-station which provides an immersion basin for taking up and heating up the reload-liquid may be employed. The electrical heating itself may be implemented by resistive heating or by applying electrically induced ultrasound (ultrasonic waves) to the reload-liquid, to name two possible implementations.

Due to reasons of health protection and effective regulations, the agent can only be contained in the reload-station at a maximum concentration that is not harmful to the patient. This maximum concentration thus normally limits the speed at which the reloading takes place. In other words, the time required to fully reload the micro-reservoirs of an appliance will be limited by that maximum concentration. The proposed approach has the great advantage of significantly reducing this time, because the diffusion of the agent into the reservoirs, will be accelerated as compared to diffusion at room temperature, in particular when employing a cap layer in the dental appliance.

Examples of the present invention will now be described in more detail with reference to the accompanying drawings, where features with corresponding technical function are referenced with same numerals even when these features differ in shape or design:
- Fig. 1: is an illustration of a fabrication of a dental appliance by thermoforming according to the state-of-the-art,
- Fig. 2: shows an illustration of fabricating a dental appliance from a thermoplastic core foil,
- Fig. 3 to 10: illustrate simplified and schematic cross-sections through dental appliances,
- Fig. 11: illustrates the effect of thermoforming on a predefined micro-reservoir in the form of a microscopic air pocket formed in the core of a dental appliance,
- Fig. 12: illustrates the effect of thermoforming on a predefined micro-reservoir in the form of a microscopic air pocket formed in the core of another dental appliance,
- Fig. 13: illustrates another possible design of a dental appliance,
- Fig. 14: illustrates a series of dental appliances according to the invention, which have been fabricated using a computer-implemented method according to the invention, and
- Fig. 15: illustrates the use of a reload-station designed for reloading or initially loading an agent into any suitable dental appliance.

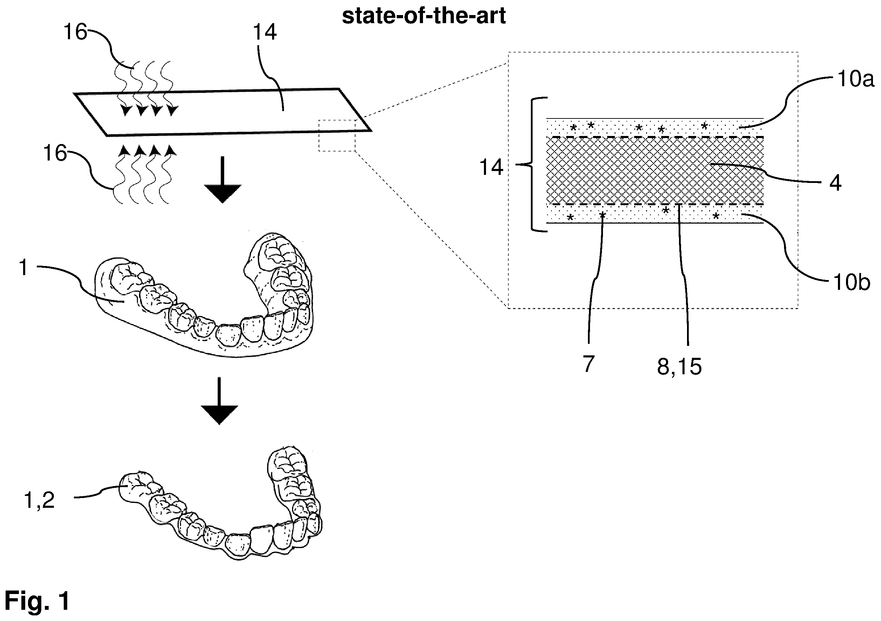

Figure 1 displays a process as known in the state-of-the-art for fabricating a dental appliance 1 from a thermoplastic foil 14. The foil 14 comprises a core 4 and two outer cap layers 10a and 10b. The layers 10a, 10b are softer than the core 4 and used to provide an improved wearing comfort for the user. Under the impact of heat and pressure 16, the appliance 1 can be thermoformed from the core foil 14 and trimmed in size afterwards.

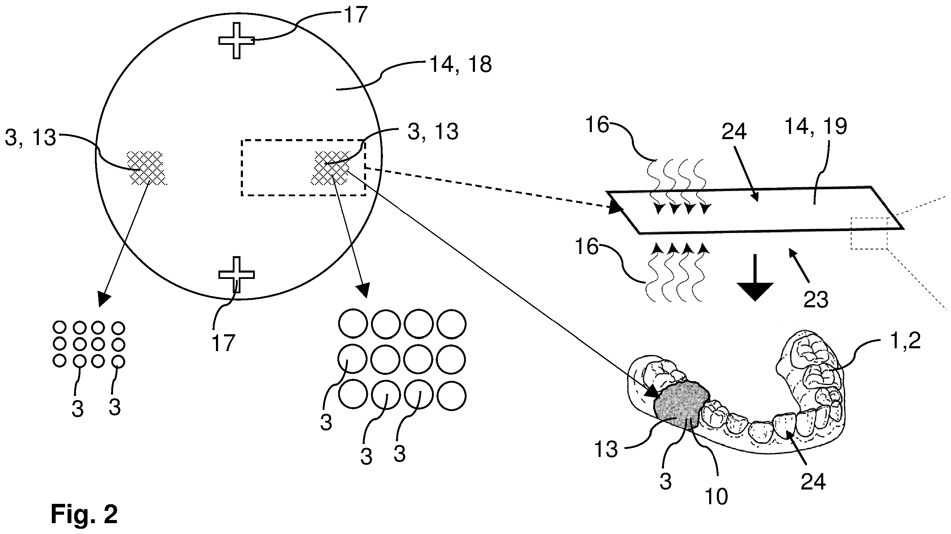

Figure 2 shows an illustration of the comparable fabrication of a first dental appliance 1 according to the invention:
Again, a thermoplastic foil 14, namely a master foil 18, is employed but prior to thermoforming, a multitude of sub-mm sized micro-reservoirs 3 are formed in the foil 14 within a particular region of interest 13 (ROI) using an ablative lasering process. The micro-reservoirs 3 are arranged as a 2D-array and the location of each individual micro-reservoir 3 is controlled by considering fiducials 17 on the master foil 18. Afterwards the master foil 18 is cut into single pieces 19, and from each of the pieces 19 a dental appliance 1 is obtained by thermoforming the piece 19. The location of the micro-reservoirs 3 within the piece 19 is thus also well-known.

As the micro-reservoirs 3 have been patterned in the outer surface 24 of the foil 14, the micro-reservoirs 3 of the final appliance 1 are facing the gums and inner oral cavity of the patient, when the patient is wearing the dental appliance 1 on his teeth. As illustrated in figure 2, arranging the micro-reservoirs 3 within a certain area of the master foil 18 has resulted in the micro-reservoirs being located in a region of interest (ROI) 13 that is located at the one-before-last molar of the patient.

The micro-reservoirs 3 in Figure 2 can be such that they are formed with the same diameters and same depth and show a diameter of less than 0.2 mm and a depth of 0.1 mm, resulting in an aspect ratio of AR = depth/diameter > 0.5.

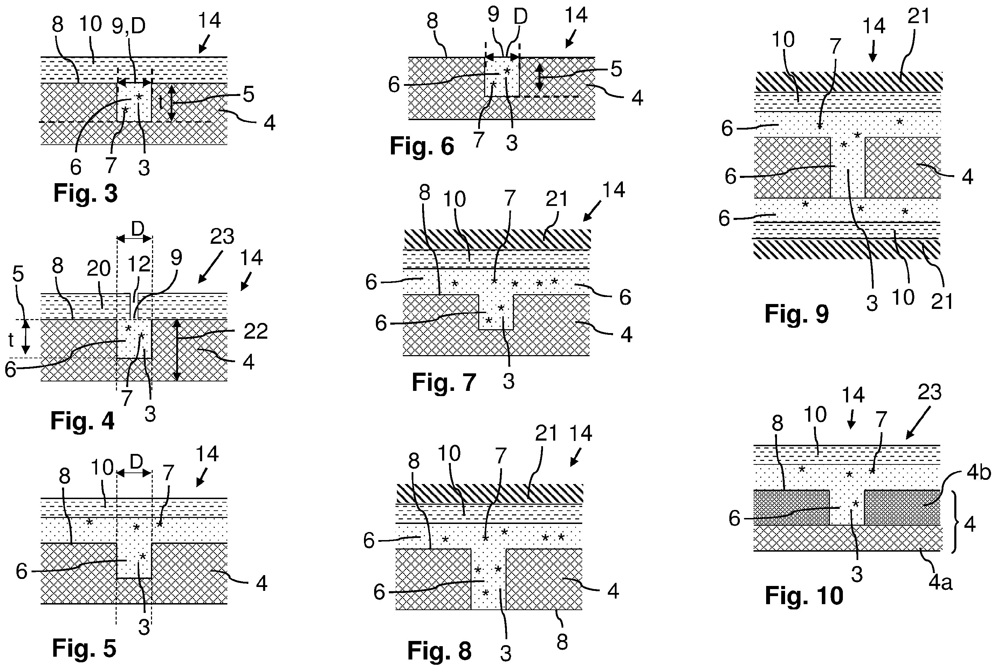

Figures 3 to 10 provide further examples of possible embodiments of dental appliances 1.

In all examples according to the invention, a material is used for the core 4 which shows a negligible diffusion rate for the agent 7, such that the core 4 can be considered impermeable for the agent 7. Shown are simplified and schematic cross-sections through the foil 14, from which the final appliance 1 will be thermoformed. Each appliance 1 will thus feature a solid core 4, in which a multitude of micro-reservoirs 3 have been patterned, although only one of these micro-reservoirs 3 is illustrated. In the shown examples, each micro-reservoir 3 is entirely filled up with a filling material 6, which is capable of storing and releasing an antimicrobial agent 7.

In the example of figure 3, the micro-reservoir 3 is formed as at dead hole, with the core 4 forming a bottom of that hole. The micro-reservoir 3 is covered and closed by a cap layer 10 which forms a diffusion barrier for the agent 7 contained in the filling material 6, because the diffusion rate of the agent 7 is much lower in the cap layer 10 as compared to the filling material 6.

Compared to the example of Figure 6, in which no such cap layer 10 is employed (such that the micro-reservoir 3 forms a micro-release-opening 9 of diameter D in the outer surface 8 of the foil 14 and hence the final core 4 of the appliance 1), the release rate of the design shown in Figure 3 will be much lower. As a result, the amount of time during which a significant amount of the agent 7 can be released from the appliance 1 will be prolonged in the example of Figure 3 as compared to Figure 6, at least when using the same materials and agent 7.

Figure 4 illustrates another possible approach, in which a dedicated release hole 12 is employed, which has been formed in the cap layer 10. In this example, the cap layer is a layer 20 that is impermeable to the agent 7. Accordingly, the agent 7 can only be loaded into the micro-reservoir 3 and released from the same through the release hole 12. The diameter of the release hole 12 thus provides convenient control of the release rate, because it defines the cross-sectional area through which the transfer of the agent 7 can happen.

The difference between the design of figure 5 compared to that of figure 3 is that the filling material 6 not only fills up the micro-reservoir 3 but also covers at least part of the outer surface of the core 4. As illustrated by the star symbols, the agent 7 can thus not only be embedded into the micro-reservoirs 3 but also in the superficial part of the filling material 6 layer just below the outer cap layer 10. It is obvious that the agent 7 can freely diffuse within the filling material 6.

In the example of Figure 7, an additional outermost layer 21 has been added as compared to the design of Figure 5. This layer 21 can swell up in water and is used for the sole purpose of improving the wearing comfort of the dental appliance 1. In this design, the cap layer 10 is therefore not an outermost layer but an inner layer off the muti-layer structure displayed.

The example of figure 8 differs from that of figure 7 in that the micro-reservoir 3 is no longer formed as a dead hole but rather as a through hole. This has the advantage that the micro-reservoir 3 can be loaded with the agent 7 from the upper and lower side.

In the more sophisticated design of figure 9, an additional cap layer 10 and additional outermost layer 21 have been added to the lower side of the structure, which may form the inner surface of the final dental appliance 1 that is resting on the teeth.

Figure 10 illustrates another possible design of a dental appliance 1 in which the core 4 is designed as a multi-layer structure with a solid inner core 4a on which an outer core layer 4b has been 3D-printed. In this design, the micro-reservoirs 3 have thus been formed in a separate outer layer 4b of the core 4 while fabricating the core 4 using an additive manufacturing process, namely 3D-printing. Afterwards, the micro-reservoirs 3 have been filled up with a filling material 6, which also covers the micro-reservoir 3. Finally, a cap layer 10 was added to form a diffusion barrier.

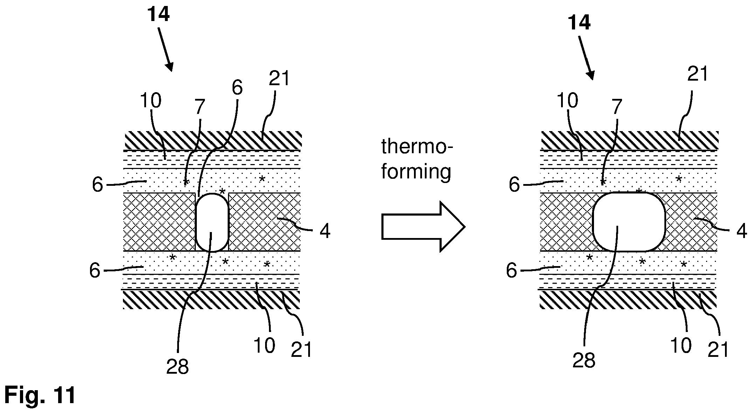

Figure 11 illustrates the example of a micro-reservoir 3 formed as an air pocket 28 with a diameter of 0.2 mm in a solid core 4 of a thermoplastic material. This was achieved simply by drilling a hole through the core 4 and applying a filling material 6 using blade coating processes on both sides of the core 4. As can be seen in figure 11 on the left, this process does not fill up the micro-reservoir 3 completely, such that said air pocket 28 remains in the core 4. After adding cap layers 10 and soft outermost layers 21 on both sides of the core foil 14, the structure is ready for thermoforming. On the right of figure 11, the result of the thermoforming is displayed. As visible, the air pocket 28 has expanded due to the elevated temperatures during thermoforming and sort of fused with the material of the core 4, which was highly softened during the thermoforming. As a result of this change in geometry, the cross-section through which diffusion can happen has been reduced leading to a lower release rate.

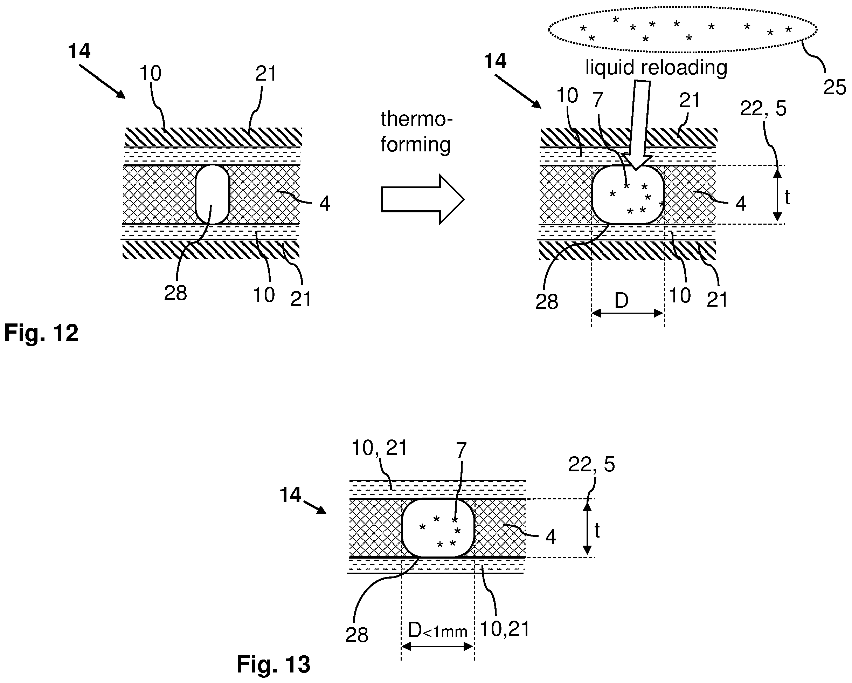

Figure 12 illustrates another design, in which an air pocket 28 is used as a micro-reservoir 3 that is embedded in the core 4 of the dental appliance 1. After thermoforming of the core foil 14, the micro-reservoir 3 has been stretched and enlarged. By immersing the dental appliance 1 into a reload-liquid 25 which contains the agent 7 in a high concentration, the agent 7 can be loaded into the micro-reservoir 3 by letting the agent 7 diffuse through the outermost layer 21 and the cap layer 10. In this case, the agent 7 and other ingredients of the reload-liquid 25 can fully replace the air in the pocket 28 until the pocket 28 / the micro-reservoir 3 is fully loaded. This process may be repeated (= reloading of the appliance 1) after the dental appliance 1 has been used by a user and has released most of the amount of agent 7 stored in the multitude of micro-reservoirs 3.

As figure 13 displays, air pockets 28 may also be used in a simple design in which the core 4 / the micro-reservoir 3 is only covered by a single outermost cap layer 10 (for example on both sides as displayed).

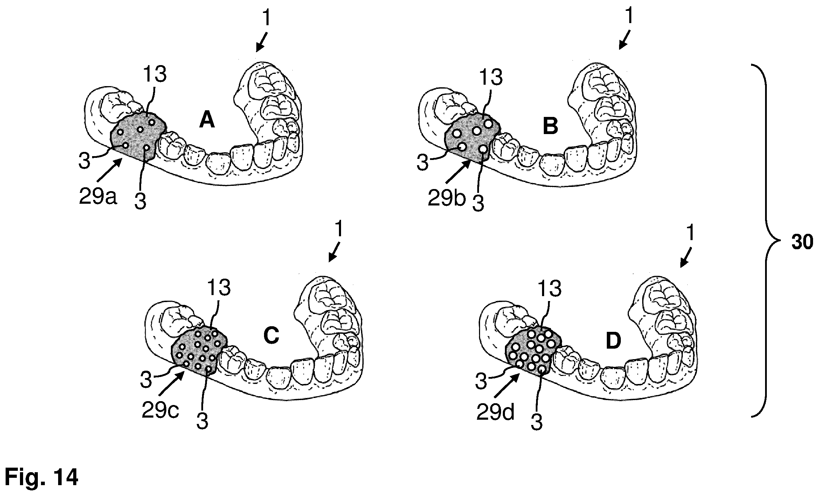

Figure 14 illustrates a series of four different dental appliances 1 A-D, which are all fabricated from the same materials using same processes and are all tailored in shape to the teeth of one patient. The appliances 1 differ, however, in the arrangement 29 of the micro-reservoirs 3 with respect to spatial density and/or individual size of the micro-reservoirs 3. As indicated (for example compare A to B), for changing the release rate and/or the maximum dosage of the agent 7 delivered by one of the appliances 1, it may be sufficient to alter a geometrical design parameter of the micro-reservoirs 3 such as their diameter (and thereby the size, volume and hence the amount of agent in mg that can be stored in each micro-reservoir 3) while maintaining a particular pattern of the 2D-arrangement of the micro-reservoirs 3.

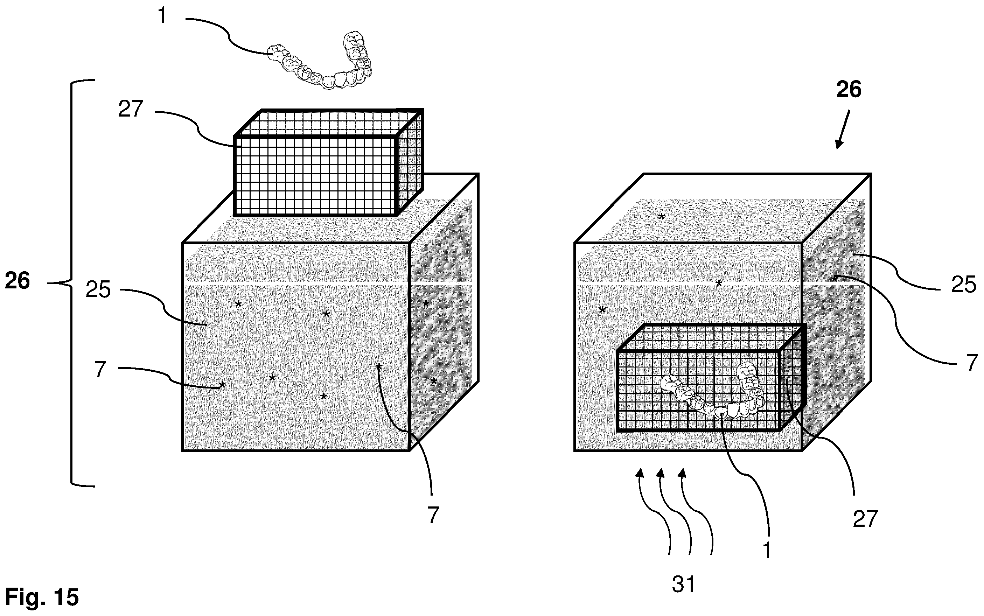

Figure 15 finally illustrates the use of a reload-station 26. As illustrated, the reload-station 26 offers an immersion basin 27 for taking up and heating up electrically a reload-liquid 25 containing the agent 7. Due to the elevated temperatures, the diffusion process is accelerated resulting in a greatly reduced reload-time.

### List of reference numerals

- 1: dental appliance
- 2: dental splint
- 3: micro-reservoir
- 4: core
- 5: depth (of 3)
- 6: filling material
- 7: agent
- 8: outer surface (of 4)
- 9: micro-release-opening (of 3 in 8)
- 10: cap layer (covering 4 and 3)
- 11: surface area (covered by 10)
- 12: release hole (formed in 10)
- 13: region of interest (on 4, in which 3 are located)
- 14: (thermoplastic) foil
- 15: interface (e.g. between 4 and 10)
- 16: heat, pressure
- 17: fiducial
- 18: master foil
- 19: piece (cut out of 18)
- 20: impermeable cap layer
- 21: outermost layer (e.g., for improving the wearing comfort of 1) - may swell up in water
- 22: thickness (of 4)
- 23: inner surface (of 1)
- 24: outer surface (of 1)
- 25: reload-liquid (containing 7)
- 26: reload-station
- 27: immersion basin
- 28: air pocket
- 29: micro-reservoir-arrangement
- 30: series of dental appliances
- 31: electrical heating (resistive heating or ultrasound)
- 32: manufacturing means
- 33: means for thermoplastic formation of micro-reservoirs
- 34: means for ablative formation of micro-reservoirs
- 35: means for additive formation of micro-reservoirs

## Claims

1. A **computer-implemented method** for producing a series (30) of different dental appliances (1), **the method comprising the following steps:**
- specifying a number of m different numerical values for at least one agent release parameter for a particular releasable agent (7) in a digital data set;
- calculating a set of m different design parameters for defining a set of m different micro-reservoir-arrangements (29), in particular using a numerical and empirical model;
- producing said series (30) of m different dental appliances (1) using manufacturing means (32),
- wherein each of the m dental appliances (1) features an arrangement (29) of micro-reservoirs (3) according to one of the m different design parameters,
- such that each of the m different dental appliances (1) meets the respective specified release parameter.

2. The computer-implemented method according to the previous claim, wherein the digital data set specifying the m different numerical values for the at least one agent release parameter comprises
- a set of patient-specific values, each being based on the specific medical need of a single patient, in particular as specified by a treatment instruction for that patient and/or
- a subset of N treatment-specific-values, each based on a specified dosage of the at least one agent to be delivered to a particular patient within a specified time interval as laid out in a treatment plan for that patient.

3. The computer-implemented method according to claim 1 or 2,
- wherein said manufacturing means (32) comprise
- means (33) for thermoplastic formation of the micro-reservoirs (3) and/or
- means (34) for ablative formation of the micro-reservoirs (3) and/or
- means (35) for additive formation of the micro-reservoirs (3),
- preferably such that by applying the method, each of the dental appliances (1) is fabricated according to a specific set of m different design parameters using said manufacturing means (32).

4. The computer-implemented method according to one of the preceding claims, wherein the digital data set comprises
- a maximum initial release rate in mg/hour and/or
- a total maximum release amount in mg over a time period of 6 hours and/or
- a maximum areal release rate in (mg/cm²) /hour.

5. The computer-implemented method according to one of the preceding claims, wherein each of the dental appliances (1) comprises a solid core (4), and
- the respective core (4) of the respective dental appliance (1) is first formed as a core foil (14) and
- a multitude of micro-reservoirs (3) are defined in an outer surface (8) of the respective core (4) using
- an ablative process, preferably an ablative laser-process, which removes the material of the core (4) at the location of each micro-reservoir (3),
or
- a forming process, such as hot embossing,
or
- an additive process, for example molding or 3D-printing of an additional outer layer (4b) of the core (4).

## Patentansprüche

1. Computerimplementiertes Verfahren zum Produzieren einer Serie (30) unterschiedlicher Dentalgeräte (1), wobei das Verfahren folgende Schritte umfasst:
- Spezifizieren einer Anzahl von m unterschiedlichen numerischen Werten für mindestens einen Wirkstoffabgabeparameter für einen bestimmten abgebbaren Wirkstoff (7) in einem digitalen Datensatz;
- Berechnen eines Satzes von m unterschiedlichen Auslegungsparametern zum Definieren eines Satzes von m unterschiedlichen Mikroreservoir-Anordnungen (29), insbesondere unter Verwendung eines numerischen und empirischen Modells;
- Produzieren der Serie (30) von m unterschiedlichen Dentalgeräten (1) unter Verwendung von Herstellungsmitteln (32),
- wobei jedes der m Dentalgeräte (1) eine Anordnung (29) von Mikroreservoirs (3) gemäß einem der m unterschiedlichen Auslegungsparameter aufweist,
- derart, dass jedes der m unterschiedlichen Dentalgeräte (1) den jeweiligen spezifizierten Abgabeparameter erfüllt.

2. Computerimplementiertes Verfahren nach dem vorhergehenden Anspruch, wobei der digitale Datensatz, der die m unterschiedlichen numerischen Werte für den mindestens einen Wirkstoffabgabeparameter spezifiziert, Folgendes umfasst
- einen Satz patientenspezifischer Werte, von denen jeder auf dem spezifischen medizinischen Bedarf eines einzelnen Patienten basiert, insbesondere wie von einer Behandlungsanweisung für diesen Patienten spezifiziert, und/oder
- einen Untersatz von N behandlungsspezifischen Werten, von denen jeder auf einer spezifizierten Dosierung des mindestens einen Wirkstoffs basiert, der einem bestimmten Patienten innerhalb eines spezifizierten Zeitintervalls wie in einem Behandlungsplan für diesen Patienten dargelegt zu verabreichen ist.

3. Computerimplementiertes Verfahren nach Anspruch 1 oder 2,
- wobei die Herstellungsmittel (32) Folgendes umfassen
- Mittel (33) zur thermoplastischen Bildung der Mikroreservoirs (3) und/oder
- Mittel (34) zur ablativen Bildung der Mikroreservoirs (3) und/oder
- Mittel (35) zur additiven Bildung der Mikroreservoirs (3),
- vorzugsweise derart, dass durch Anwenden des Verfahrens jedes der Dentalgeräte (1) gemäß einem spezifischen Satz von m unterschiedlichen Auslegungsparameter unter Verwendung der Herstellungsmittel (32) gefertigt wird.

4. Computerimplementiertes Verfahren nach einem der vorhergehenden Ansprüche, wobei der digitale Datensatz Folgendes umfasst
- eine maximale anfängliche Abgaberate in mg/Stunde und/oder
- eine maximale Gesamtabgabemenge in mg über einen Zeitraum von 6 Stunden und/oder
- eine maximale flächenbezogene Abgaberate in (mg/cm²) / Stunde.

5. Computerimplementiertes Verfahren nach einem der vorhergehenden Ansprüche, wobei jedes der Dentalgeräte (1) einen festen Kern (4) umfasst und
- der jeweilige Kern (4) des jeweiligen Dentalgeräts (1) zuerst als eine Kernfolie (14) geformt wird und
- eine Vielzahl von Mikroreservoirs (3) in einer Außenfläche (8) des jeweiligen Kerns (4) definiert wird unter Verwendung
- eines ablativen Prozesses, vorzugsweise eines ablativen Laserprozesses, der das Material des Kerns (4) an dem Ort eines jeden Mikroreservoirs (3) entfernt,
oder
- eines Formprozesses, wie zum Beispiel Heißprägen,
oder
- eines additiven Prozesses, wie zum Beispiel Gießen oder 3D-Drucken einer zusätzlichen Außenschicht (4b) des Kerns (4).

## Revendications

1. **Procédé implémenté par ordinateur** pour produire une gamme (30) d'appareils dentaires (1) différents, **le procédé comprenant les étapes suivantes :**
- la spécification d'un nombre de m valeurs numériques différentes pour au moins un paramètre de libération d'agent pour un agent libérable (7) particulier dans un ensemble de données numériques ;
- le calcul d'un ensemble de m paramètres de conception différents pour définir un ensemble de m agencements de microréservoirs (29) différents, en particulier à l'aide d'un modèle numérique et empirique ;
- la production de ladite gamme (30) de m appareils dentaires (1) différents à l'aide de moyens de fabrication (32),
- dans lequel chacun des m appareils dentaires (1) présente un agencement (29) de microréservoirs (3) selon l'un des m paramètres de conception différents,
- de telle sorte que chacun des m appareils dentaires (1) différents satisfait le paramètre de libération spécifié respectif.

2. Procédé implémenté par ordinateur selon la revendication précédente, dans lequel l'ensemble de données numériques spécifiant les m valeurs numériques différentes pour l'au moins un paramètre de libération d'agent comprend
- un ensemble de valeurs spécifiques au patient, chacune étant basée sur le besoin médical spécifique d'un seul patient, en particulier tel que spécifié par une instruction de traitement pour ce patient et/ou
- un sous-ensemble de N valeurs spécifiques au traitement, chacune étant basée sur une posologie spécifiée de l'au moins un agent à délivrer à un patient particulier au sein d'un intervalle de temps spécifié, tel qu'exposé dans un plan de traitement pour ce patient.

3. Procédé implémenté par ordinateur selon la revendication 1 ou 2,
- dans lequel lesdits moyens de fabrication (32) comprennent
- des moyens (33) pour la formation thermoplastique des microréservoirs (3) et/ou
- des moyens (34) pour la formation ablative des microréservoirs (3) et/ou
- des moyens (35) pour la formation additive des microréservoirs (3),
- de préférence de telle sorte que, en appliquant le procédé, chacun des appareils dentaires (1) soit fabriqué selon un ensemble spécifique de m paramètres de conception différents à l'aide desdits moyens de fabrication (32).

4. Procédé implémenté par ordinateur selon l'une des revendications précédentes, dans lequel l'ensemble de données numériques comprend
- un taux de libération initial maximal en mg/heure et/ou
- une quantité totale maximale de libération en mg sur une période de 6 heures et/ou
- un taux de libération maximal de superficie en (mg/cm²)/heure.

5. Procédé implémenté par ordinateur selon l'une des revendications précédentes, dans lequel chacun des appareils dentaires (1) comprend un noyau (4) solide, et
- le noyau (4) respectif de l'appareil dentaire (1) respectif est d'abord formé comme une feuille de noyau (14) et
- une multitude de microréservoirs (3) sont définis dans une surface externe (8) du noyau (4) respectif en utilisant
- un processus ablatif, de préférence un processus laser ablatif, qui enlève le matériau du noyau (4) à l'emplacement de chaque microréservoir (3),
ou
- un processus de formage, tel qu'un gaufrage à chaud,
ou
- un processus additif, par exemple un moulage ou une impression 3D d'une couche externe supplémentaire (4b) du noyau (4).
